Europäisches Patentamt

European Patent Office

Office européen des brevets

(19)

(11) Publication number: **0 242 941 B1**

(12) **EUROPEAN PATENT SPECIFICATION**

(45) Date of publication of patent specification: **29.07.92** (51) Int. Cl.5: **A61L 9/015**

(21) Application number: **87300766.0**

(22) Date of filing: **29.01.87**

The file contains technical information submitted after the application was filed and not included in this specification

(54) **Process and apparatus for the deodorization of air.**

(30) Priority: **18.04.86 GB 8607061**

(43) Date of publication of application:
**28.10.87 Bulletin 87/44**

(45) Publication of the grant of the patent:
**29.07.92 Bulletin 92/31**

(84) Designated Contracting States:
**BE DE FR GB NL SE**

(56) References cited:
**EP-A- 0 057 624**
**DE-A- 3 121 686**
**DE-A- 3 405 142**
**FR-A- 2 543 831**
**US-A- 4 156 652**

**PATENT ABSTRACTS OF JAPAN, vol. 2, no. 122 (C-78)[2500], 13th October 1978; & JP-A-53 89 875 (MITSUBISHI DENKI K.K.) 08-08-1978**

(73) Proprietor: **Electricity Association Services Limited**
**30 Millbank**
**London SW1P 4RD(GB)**

(72) Inventor: **Barker, Ronald**
**16 Woodfall Close**
**Little Neston South Wirral, L64 4EA(GB)**
Inventor: **Jones, Andrew Robert**
**Mintaka 14 Rhyddyn Hill**
**Caergwrle Wrexham, Clwyd, LL12 9EB(GB)**

(74) Representative: **Allard, Susan Joyce et al**
**BOULT, WADE & TENNANT, 27 Furnival Street**
**London EC4A 1PO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**Description**

The present invention relates to a process for the removal and destruction of malodorant compounds from an air stream by gas absorption combined with ultra-violet radiation and ozone, and to apparatus for carrying out this process.

Odour control can be attempted by a number of methods such as thermal combustion (incineration), catalytic combustion, adsorption by a solid and dilution, in addition to the gas-liquid absorption. Combustion is obviously very expensive in fuel and adsorption and dilution not very effective. Gas-liquid absorption, which is commonly referred to in the art as gas scrubbing is the most common way of removing malodorant compounds (at not too high concentrations) from large volume flow rates of air. This technique is relatively low in running costs and is capable, under the right conditions, of reducing the concentration of malodorant compounds down to very low levels. Normally the gas scrubbing liquid is in the form of a spray and is passed counter current to the air flow. A packed bed or a plate bed may be incorporated into the gas scrubbing apparatus to improve gas-liquid mixing and mass transfer. Water is the scrubbing liquid which is generally used, but even if the malodorant compound is very soluble in water, very little recycling of the water can be carried out before there is a fall-off in efficiency. A system in which there is a single pass of water through the incoming air would overcome this problem but would give rise to a large quantity of contaminated water, with problems of its disposal. Therefore a reactant is usually added to the water to decompose the malodorant compound and this enables the scrubbing liquid to be recycled a number of times. Furthermore if there is appreciable reaction between the reactant dissolved in the scrubbing liquid and the malodorant compound in the gas phase then mass transfer of the malodorant compound (or product) from the gas to the liquid phase will be increased; this will be particularly marked if the solubility of the original malodorant compound in water is low.

The most common type of reactant which is added to the scrubbing liquid is an oxidant, since oxidation usually produces compounds which are more soluble in water than the original compounds and less odorous. The oxidant most commonly used in gas scrubbers is sodium hypochlorite, because it is cheap. There are, however, a number of disadvantages to its use. Several stage scrubbers have to be used to try to cover the whole range of malodorant compounds, there is a tendency for chlorine gas to be given off (particularly at low pH), and chlorination can take place (rather than oxidation) which can produce very malodorant compounds (e.g. amines on chlorination produce chloramines) and at the best chlorinated organic compounds which are considered undesirable from an environmental point of view.

There is therefore a need for an oxidant which does not give rise to undesirable products and which can react with a wide range of malodorant compounds. Ozone and hydrogen peroxide are two oxidants which both fulfil the first criterion and ozone fulfils the second criterion better than hydrogen peroxide.

Ozone has found use as an oxidant in gas scrubbing systems but it does not react sufficiently quickly with some classes of malodorant compounds, e.g. some amines, carboxylic acids, ketones and aldehydes, to be effective and it is not able to oxidise any organic compound completely to form carbon dioxide and water. Because of this there is likely to be a build up of carboxylic acids (giving a decrease in pH) in the scrubbing liquid.

JP-A-5389875 describes a process for removing malodorant compounds by treating humidified air containing malodorant compounds by mixing the humidified air with ozonized air and chlorine, and thereafter subjecting the gaseous mixture to irradiation with ultraviolet irradiation.

We have now found that if ozone is used as an oxidant in liquid gas scrubbing systems, for removing malodorant compounds from air, in combination with the ultra-violet (U.V.) irradiation of the scrubbing liquid, all of the malodorant compounds are totally oxidised and thus removed from the air stream.

Accordingly, the present invention provides a process for the removal of malodorant compounds from air, which process comprises ozonating an aqueous liquid, irradiating the ozonated aqueous liquid with ultra-violet radiation having a wavelength in the range of from 220 to 300 nm and thereafter contacting the ozonated aqueous liquid with the air stream containing the malodorant compounds.

The ozone dissolved in the aqueous liquid is converted by the ultra-violet radiation into hydroxyl radicals by the reaction sequence

$$O_3 + h\nu \rightarrow O_2 + O^*$$
$$O^* + H_2O \rightarrow 2OH$$

where $O^*$ is an excited oxygen atom formed provided that the ultra-violet radiation has a wavelength $<334$ nm. These hydroxyl radicals are reactive towards almost any organic compounds, compared to the limited reactivity of ozone. The process of the present invention can therefore be used to treat any malodorant compound in an air stream and because the U.V./ozone combination is such a strongly oxidising combination all of the compounds absorbed into the aqueous liquid will be oxidised to carbon dioxide, water etc., thus leaving the aqueous scrubbing liquid clean and long lasting as compared to the scrubbing liquid in a conventional gas scrubber.

The aqueous liquid used in the process of the present invention is preferably water, although it will be understood that it may be advantageous, for the treatment of air streams contaminated with certain malodorant compounds, to use a weakly acid or weakly alkaline aqueous scrubbing liquid.

The aqueous liquid is preferably ozonated so that the ozone concentration is in the range of from 1 to 10 ppm, preferably 6 to 10 ppm.

The ultra-violet radiation is in the range of from 220 to 300 nm. A low pressure mercury lamp which has its main emission at 254 nm is a particularly suitable source of ultra-violet radiation because this emission matches very well the absorption spectrum of ozone in water, thus leading to the efficient conversion of input energy to hydroxyl radicals formed. It is preferred that the ozonated aqueous liquid is uniformly irradiated with ultra-violet radiation so that hydroxyl radicals are formed uniformly throughout the liquid.

The following Table gives a comparision of the reaction rates of ozone and hydroxyl radicals with various different classes of compounds.

| COMPOUND | $k$ in 1 mole$^{-1}$ s$^{-1}$ | |
|---|---|---|
| | $O_3$ | OH |
| Olefins | $1$ to $450 \times 10^3$ | $10^9$ to $10^{11}$ |
| S containing organics | $10^3$ to $1.6 \times 10^3$ | $10^9$ to $10^{10}$ |
| Phenols | $10^3$ | $10^9$ |
| N containing organics | $10$ to $10^2$ | $10^8$ to $10^{10}$ |
| Aromatics | $1$ to $10^2$ | $10^8$ to $10^{10}$ |
| Acetylenes | $50$ | $10^8$ to $10^9$ |
| Aldehydes | $10$ | $10^9$ |
| Ketones | $1$ | $10^9$ to $10^{10}$ |
| Alcohols | $10^{-2}$ to $1$ | $10^8$ to $10^9$ |
| Alkanes | $10^{-2}$ | $10^6$ to $10^9$ |
| Carboxylic acids | $10^{-3}$ to $10^{-2}$ | $10^7$ to $10^9$ |

The present invention also includes within its scope a gas scrubber for the removal of malodorant compounds from air which comprises a scrubbing vessel, means to ozonate an aqueous scrubbing liquid with ultra-violet radiation, means to circulate the scrubbing liquid through the vessel and means to introduce the malodorous air into the vessel and bring it into contact with the scrubbing liquid.

Preferably the aqueous scrubbing liquid is contained in a sump in the scrubbing vessel and ozone is introduced into the liquid in order to ozonate it. The aqueous scrubbing liquid is generally circulated from the sump by means of a pump to a spray head which includes a plurality of spray nozzles. A charcoal filter to remove excess ozone from the treated air may be positioned above the spray head. One or more anti-mist traps may also be positioned immediately above the spray head to prevent water being carried over from the spray section. Preferably the malodorous air stream will be introduced into the bottom of the scrubbing vessel so that it meets the aqueous scrubbing liquid counter currently. However, in some instances co-current operation may be preferred.

The gas scrubbing apparatus of the present invention may also include therein a packed bed or a plate bed to improve gas-liquid mixing and mass transfer.

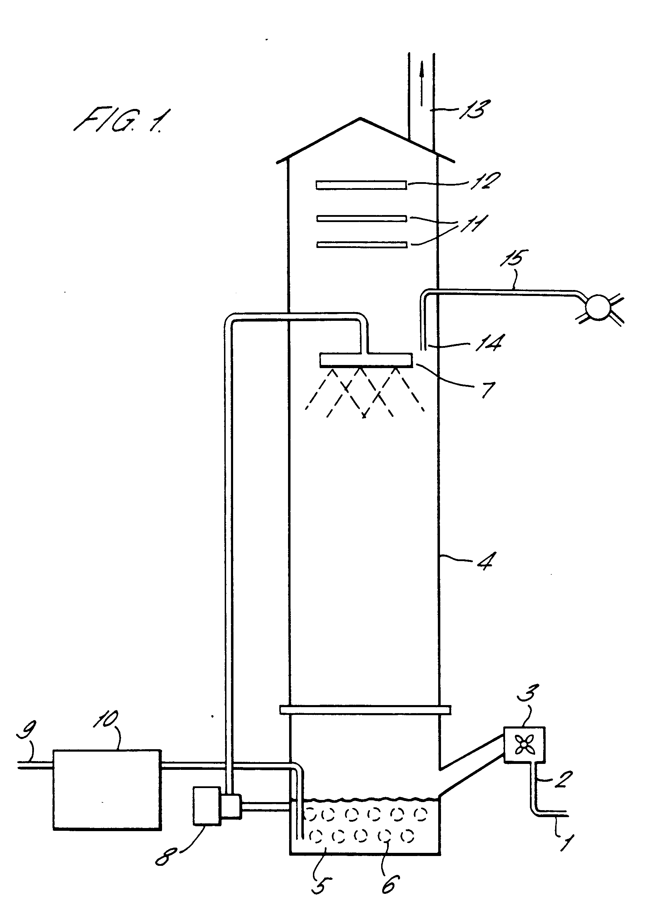

To illustrate how the present invention may be carried into effect reference is made, by way of example only, to Figure 1 of the accompanying drawings which illustrates a gas scrubber for the treatment of malodorous air.

Referring to Figure 1, a malodorant air stream 1 is passed along pipe 2 and via fan 3, into a gas scrubber 4. The gas scrubber 4 consists of a sump 5, which contains eleven 15 watt low pressure mercury lamps 6, and an H-section spray head 7 which contains five spray heads having holes of 3 to 4 mm in diameter and angled to produce jets with a circular motion. The scrubbing liquid, generally water, is continuously pumped from the sump 5 by a pump 8 to the spray head 7, and thus recirculated, at a flow-rate of 50 l min$^{-1}$.

Ozone is generated from bottled oxygen gas (with no drying) fed along pipe 9 to an ozoniser 10 and fed into the sump 5 via a diffuser bar. An oxygen flow-rate of 600 l hr$^{-1}$ produces ozone yields of about 30

g ozone $M^{-3}$.

There are two anti-mist traps 11 immediately above the spray head 7 to minimise the carry over of water from the spray section. An activated charcoal bed 12 of up to 12 cm depth is used to remove any excess ozone from the exhaust gas of the scrubber. Treated air 13 leaves from the top of the scrubber. The contact of the malodorous air and the scrubbing liquid in the gas scrubber is counter current.

The fan 3 can produce an air flow rate of 3700 l $min^{-1}$ but when the anti-mist traps 11 and the charcoal bed 12 are installed the effective air throughput is reduced to 1200 l $min^{-1}$. The volume of the scrubber liquid in the sump 5 is 15 l and of the spray section is about 100 l. The gas scrubber is constructed from unplasticised polyvinylchloride.

The total power consumption is 1240 watts, made up of : ozoniser - 500 watts; lamps - 440 watts; fan -100 watts; pump - 200 watts.

The present invention will be further described with reference to the following Example.

EXAMPLE

A gas scrubber as described with reference to Figure 1 of the accompanying drawings was used to treat malodorant air from processes for the manufacture of 4-chloro-2-methylphenoxyacetic acid (MCPA) and 2,4-dichloro-phenoxyacetic acid (2,4-D). A malodorous air stream was extracted from a $10M^3$ reaction vessel in which the hydrolysis of the sodium salts of MCPA or 2,4-D to the free acids was carried out using sulphuric acid. The odour thresholds of the main compounds involved are given below:-

| Compound | Odour threshold (ppm/weight) |
|---|---|
| Chlorophenols | 0.005 |
| 2,4-D | 3.1 |

As the impurity level of the chlorophenol was above 1.5% the odour was essentially that of chlorophenol, rather than 2,4-D.

Two types of experiment were carried out. Samples of the air stream from the above reaction vessel before the scrubber and after the scrubber (but before the charcoal filter) were bubbled into water and analysed for chlorophenol and total organic content by liquid chromatography (l.c.). Samples of the scrubber solution were also analysed for chlorophenol by a colorimetric technique and total organic carbon (TOC) by a TOC meter.

The following Table gives the results of extensive air sampling experiments which were carried out over periods of from 10 to 30 minutes, when the odour was considered to be at its maximum. The air flow rate was either 1200 or 2000 L $min^{-1}$. These results show that there is no systematic effect of the chlorophenol concentration, except possibly at the highest valves. There are small but perceptible decreases in the destruction of chlorophenol as the ozone concentration is decreased or the UV radiation is switched off.

4

| UV | $O_3$ g $M^{-3}$ | CHLOROPHENOL CONC, ug l$^{-1}$ in air | % Destruction | $\log(\frac{100}{\% \text{ rem}})$ | mean log |
|---|---|---|---|---|---|
| YES | 30 | 7.02 | 99.69 | 2.51 | 2.60 ± 0.34 |
| YES | 30 | 16.3 | 98.5 | 1.82 | |
| YES | 30 | 20.5 | 99.88 | 2.92 | |
| YES | 30 | 48.8 | 99.72 | 2.55 | |
| YES | 30 | 71.6 | 99.68 | 2.49 | |
| YES | 30 | 179 | 99.94 | 3.22 | |
| YES | 30 | 361 | 99.75 | 2.60 | |
| YES | 30 | 382 | 99.94 | 3.22 | |
| YES | 30 | 700 | 99.21 | 2.10 | |
| YES | 10 | 14.6 | 99.67 | 2.48 | 2.32 ± 0.17 |
| YES | 10 | 10.6 | 99.30 | 2.15 | |
| NO | 30 | 15.6 | 99.76 | 2.62 | 2.33 ± 0.29 |
| NO | 30 | 16.2 | 99.09 | 2.04 | |
| NO | 10 | 17.5 | 99.70 | 2.52 | 2.02 ± 0.25 |
| NO | 10 | 28.8 | 99.04 | 2.02 | |
| NO | 10 | 12.2 | 98.37 | 1.79 | |
| NO | 10 | 19.5 | 98.16 | 1.74 | |

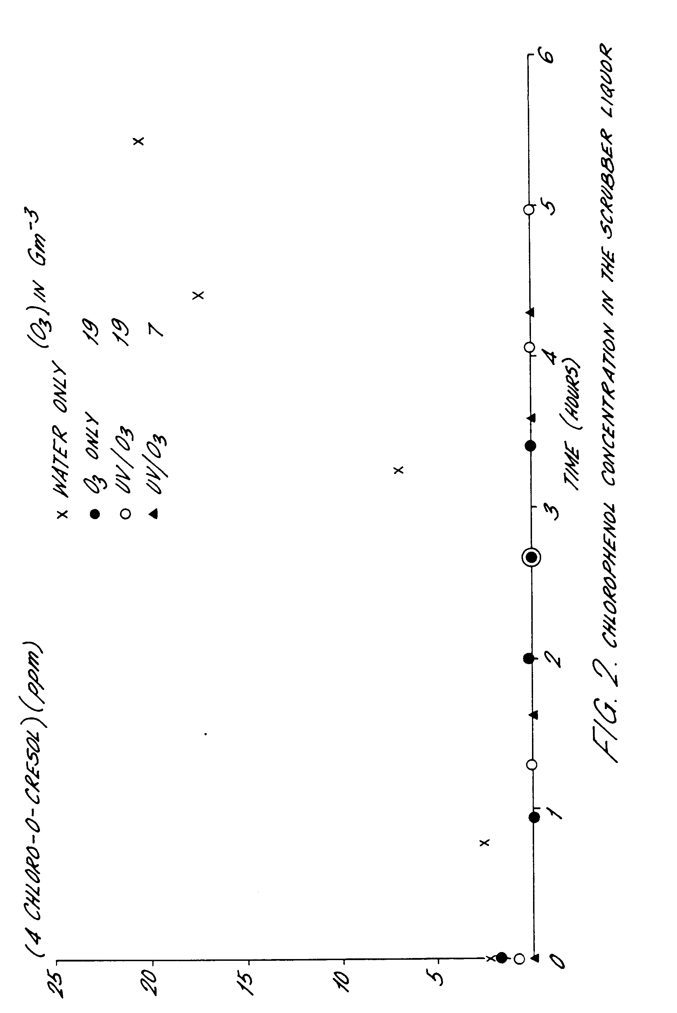

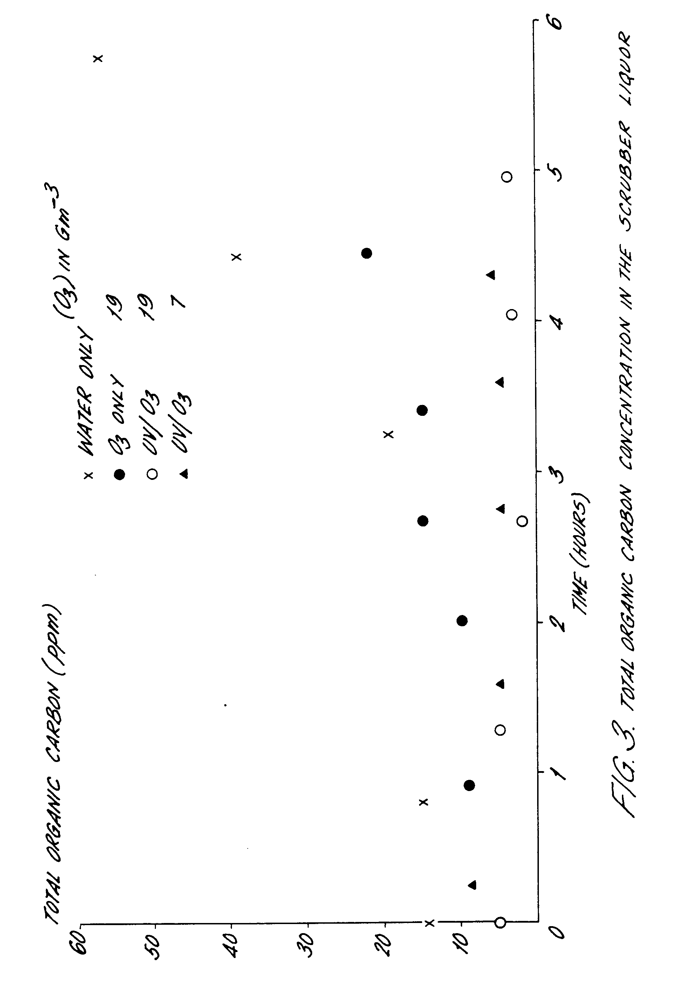

The results of the analyses of the scrubbing liquid are shown in Figures 2 and 3. In Figure 2 there was a steady build up of the chlorophenol (here 4-chloro-o-cresol) with water alone and no chlorophenol when either ozone or UV/ozone were used. Figure 3 shows again that with water alone there was a steady build up of TOC in the scrubbing liquid, with ozone alone there was a slower build up and with UV/ozone there was virtually no build up of TOC over the 5-6 hour period. It can be seen that there was a slight effect of the concentration of ozone with the UV/ozone technique, more effective destruction of the organic compounds being achieved at the higher ozone concentration.

### Claims

1. A process for the removal of malodorant compounds from air, which process comprises ozonating an aqueous liquid, irradiating the ozonated aqueous liquid with ultra-violet radiation having a wavelength in the range of from 220 to 300 nm and thereafter contacting the ozonated aqueous liquid with the air stream containing the malodorant compounds.

2. A process as claimed in claim 1 wherein the aqueous liquid is water.

3. A process as claimed in claim 1 or claim 2 wherein the aqueous liquid is ozonated to provide an ozone concentration in the range of from 1 to 10 ppm, preferably 6 to 10 ppm.

4. A process as claimed in any one of the preceding claims wherein the source of ultra-violet radiation is a low pressure mercury vapour lamp.

5. A process as claimed in any one of the preceding claims wherein the ozonated aqueous liquid is uniformly irradiated with ultra-violet radiation.

6. A gas scrubber for the removal of malodorant compounds from air which comprises a scrubbing vessel, means to ozonate an aqueous scrubbing liquid, means to irradiate the ozonated aqueous scrubbing liquid with ultra-violet radiation, means to circulate the scrubbing liquid through the vessel and means to introduce the malodorous air into the vessel and bring it into contact with the scrubbing liquid.

7. A gas scrubber as claimed in claim 6 wherein the aqueous scrubbing liquid is contained in a sump in the scrubbing vessel and a plurality of low pressure mercury lamps are contained in the sump to irradiate the ozonated scrubbing liquid.

8. A gas scrubber as claimed in claim 7 wherein the scrubbing liquid is circulated from the sump to a spray head contained in the scrubbing vessel.

9. A gas scrubber as claimed in claim 8 wherein a charcoal filter is positioned above the spray head to remove excess ozone from the air stream.

10. A gas scrubber as claimed in claim 8 or claim 9 wherein a mist trap is positioned immediately above the spray head.

11. A gas scrubber as claimed in any one of claims 7 to 10 wherein the means to introduce malodorous air into the vessel is arranged so that the contact of the air with the scrubbing liquid is counter current.

### Revendications

1. Procédé pour l'élimination de composés malodorants de l'air, procédé qui comprend l'ozonisation d'un liquide aqueux, l'irradiation du liquide aqueux ozonisé avec une radiation ultraviolette ayant une longueur d'onde dans la plage allant de 220 à 300 nm et après quoi, mise en contact du liquide aqueux ozonisé avec le courant d'air contenant les composés malodorants.

2. Procédé selon la revendication 1, dans lequel le liquide aqueux est l'eau.

3. Procédé selon la revendication 1 ou 2, dans lequel le liquide aqueux est ozonisé pour fournir une concentration en ozone dans la gamme allant de 1 à 10 ppm, de préférence de 6 à 10 ppm.

4. Procédé selon l'une quelconque des revendications précédentes, dans lequel la source de radiation ultraviolette est une lampe à vapeur de mercure à basse pression.

5. Procédé selon l'une quelconque des revendications précédentes dans lequel le liquide aqueux ozonisé est uniformément irradié avec une radiation ultraviolette.

6

**6.** Epurateur de gaz pour l'élimination de composés malodorants de l'air qui comprend une cuve de lavage, un moyen pour ozoniser un liquide de lavage aqueux, un moyen pour irradier le liquide de lavage aqueux ozonisé avec une radiation ultra-violette, un moyen pour faire circuler le liquide de lavage à travers la cuve et un moyen pour introduire l'air malodorant à l'intérieur de la cuve et l'amener en contact avec le liquide de lavage.

**7.** Epurateur de gaz selon la revendication 6, dans lequel le liquide de lavage aqueux est contenu dans une citerne dans la cuve de lavage et un ensemble de lampes à mercure à basse pression est contenu dans la citerne pour irradier le liquide de lavage ozonisé.

**8.** Epurateur de gaz, selon la revendication 7, dans lequel le liquide de lavage circule de la citerne à une tête de pulvérisation contenue dans la cuve de lavage.

**9.** Epurateur de gaz selon la revendication 6, dans lequel un filtre à charbon de bois est placé au dessus de la tête de pulvérisation pour éliminer l'excès d'ozone du courant d'air.

**10.** Epurateur de gaz selon la revendication 8 ou 9, dans lequel un piège à buée est placé immédiatement au dessus de la tête de pulvérisation.

**11.** Epurateur de gaz, selon l'une quelconque des revendications 7 à 10, dans lequel le moyen pour introduire l'air malodorant à l'intérieur de la cuve est disposé de façon que le contact de l'air avec le liquide de lavage se fasse à contre courant.

**Patentansprüche**

**1.** Verfahren zur Beseitigung übelriechender Verbindungen aus Luft, bei dem man eine wäßrige Flüssigkeit ozonisiert, die ozonisierte wäßrige Flüssigkeit mit ultravioletter Strahlung mit einer Wellenlänge in dem Bereich von 220 bis 303 nm bestrahlt und danach die ozonisierte wäßrige Flüssigkeit mit dem die übelriechenden Verbindungen enthaltenden Luftstrom in Kontakt bringt.

**2.** Verfahren nach Anspruch 1, wobei die wäßrige Flüssigkeit Wasser ist.

**3.** Verfahren nach Anspruch 1 oder 2, wobei aie wäßrige Flüssigkeit zur Erzielung einer Ozonkonzentration in dem Bereich von 1 bis 10 ppm, vorzugsweise 6 bis 10 ppm, ozonisiert wird.

**4.** Verfahren nach wenigstens einen der vorhergehenden Ansprüche, wobei die Quelle der ultravioletten Strahlung eine Quecksilberniederdrucklampe ist.

**5.** Verfahren nach wenigstens einem der vorhergehenden Ansprüche, wobei man die wäßrige Flüssigkeit gleichmäßig mit ultravioletter Strahlung bestrahlt.

**6.** Gaswascher zur Beseitigung übelriechender Verbindungen aus Luft, umfassend einen Waschbehälter, Mittel zum Ozonisieren einer wäßrigen Waschflüssigkeit, Mittel zur Bestrahlung der ozonisierten wäßrigen Waschflüssigkeit mit ultravioletter Strahlung, Mittel zur Bewirkung einer Zirkulation der Waschflüssigkeit in dem Behälter und Mittel, die dazu vorgesehen sind, die übelriechende Luft in den Behälter einzubringen und mit der Waschflüssigkeit in Kontakt zu bringen.

**7.** Gaswascher nach Anspruch 6, wobei die wäßrige Waschflüssigkeit in einem Sumpf in dem Waschbehälter enthalten ist und wobei ferner eine Vielzahl von Quecksilberniederdruchlampen zur Bestrahlung der Ozonisierten Waschflüssigkeit in dem Sumpf enthalten sind.

**8.** Gaswascher nach Anspruch 7, wobei die Waschflüssigkeit von dem Sumpf zu einem in dem Waschbehälter enthaltenen Sprühkopf umgewälzt wird.

**9.** Gaswascher nach Anspruch 8, wobei ein Kohlefilter oberhalb des Sprühkopfes positioniert ist, um überschüssiges Ozon aus dem Luftstrom zu entfernen.

**10.** Gaswascher nach Anspruch 8 oder 9, wobei unmittelbar oberhalb des Sprühkopfes ein Tropfenfänger positioniert ist.

11. Gaswascher nach wenigstens einem der Ansprüche 7 bis 10, wobei die Mittel zum Einbringen der übelriechenden Luft in den Behälter derart eingerichtet sind, daß der Kontakt der Luft mit der Waschflüssigkeit im Gegenstrom stattfindet.

FIG. 1.

(4 CHLORO-O-CRESOL)(ppm)

$(O_3)$ IN $Gm^{-3}$

x WATER ONLY
● $O_3$ ONLY     19
○ UV/$O_3$     19
▲ UV/$O_3$     7

TIME (HOURS)

FIG. 2. CHLOROPHENOL CONCENTRATION IN THE SCRUBBER LIQUOR

FIG. 3. TOTAL ORGANIC CARBON CONCENTRATION IN THE SCRUBBER LIQUOR